(19) Europäisches Patentamt
European Patent Office
Office européen des brevets

(11) EP 0 553 009 B1

(12) FASCICULE DE BREVET EUROPEEN

(45) Date de publication et mention de la délivrance du brevet:
**04.06.1997 Bulletin 1997/23**

(51) Int. Cl.$^6$: **B01J 31/02**, B01J 27/125

(21) Numéro de dépôt: **93400095.1**

(22) Date de dépôt: **15.01.1993**

# (54) Catalyseur d'alkylation de paraffines

Katalysator für die Alkylierung von Olefinen

Paraffins alkylation catalyst

(84) Etats contractants désignés:
**DE GB IT**

(30) Priorité: **23.01.1992 FR 9200802**
**24.06.1992 FR 9207859**

(43) Date de publication de la demande:
**28.07.1993 Bulletin 1993/30**

(73) Titulaire: **INSTITUT FRANCAIS DU PETROLE**
**92502 Rueil-Malmaison (FR)**

(72) Inventeurs:
- **Benazzi, Eric**
  **F-78360 Montesson (FR)**
- **Hirschauer, André**
  **F-78360 Montesson (FR)**
- **Joly, Jean-Francois**
  **F-75003 Paris (FR)**
- **Olivier, Hélène**
  **F-92500 Rueil Malmaison (FR)**
- **Bernhard, Jean-Yves**
  **F-91540 Mennecy (FR)**

(56) Documents cités:
**EP-A- 0 126 951**
**EP-A- 0 443 167**
**WO-A-90/00534**
**FR-A- 2 626 572**
**US-A- 2 337 014**
**US-A- 4 908 122**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 553 009 B1

Printed by Rank Xerox (UK) Business Services
2.14.7/3.4

## Description

La présente invention concerne un catalyseur comprenant un support poreux organique ou minéral et au moins un mélange constitué d'au moins un halogénure d'aluminium et/ou de bore et d'au moins un halogénure d'ammonium quaternaire et/ou halohydrate d'amine, et son utilisation en alkylation catalytique d'isobutane et/ou d'isopentane au moyen d'au moins une oléfine comprenant de 2 à 6 atomes de carbone par molécule, c'est-à-dire d'une oléfine $C_2$-$C_6$, qui permet d'obtenir des composés paraffiniques à haut degré de ramification et à haut indice d'octane.

On sait que, pour alimenter les moteurs à combustion interne et à allumage commandé, et notamment les moteurs à taux de compression élevé, il est particulièrement intéressant de disposer de carburants à haut indice d'octane, c'est-à-dire constitués essentiellement par des hydrocarbures paraffiniques fortement ramifiés. L'alkylation des isoparaffines (isobutane et/ou isopentane) par les oléfines contenant de 2 à 6 atomes de carbone par molécule permet d'obtenir de tels produits. Cette réaction nécessite la mise en oeuvre de catalyseurs très acides, dans le but notamment de réduire les réactions parasites telles que les réactions d'abstraction d'hydrure de l'oléfine et de polymérisation, qui fournissent des hydrocarbures peu ramifiés de faible indice d'octane et des hydrocarbures insaturés, les réactions de craquage et les réactions de dismutation.

Les procédés industriels existants pour la production d'hydrocarbures par alkylation de l'isobutane et/ou de l'isopentane par les oléfines $C_2$-$C_6$ utilisent soit l'acide sulfurique soit l'acide fluorhydrique comme catalyseur. Dans ces procédés le catalyseur acide constitue une phase liquide qui est mise en contact avec le mélange liquide isoparaffine(s)-oléfine(s) pour former une émulsion. Ces procédés sont coûteux et posent d'importants problèmes vis-à-vis de la sécurité des personnes et vis-à-vis de l'environnement. Afin de remédier à ces problèmes, des systèmes catalytiques différents des acides sulfurique et fluorhydrique en phase liquide ont été recherchés.

Pour catalyser les réactions d'alkylation des isoparaffines par les oléfines, on a déjà proposé de mettre au point des catalyseurs acides à partir de nombreux solides acides de natures différentes. Parmi les familles de catalyseurs acides on peut citer les tamis moléculaires (voir par exemple US-A-3.236.762, US-A-3.251.902, US-A-3.644.565, US-A-4.377.721, US-A-4.384.161 et US-A-4.300.015), les résines macroréticulaires éventuellement associées avec $BF_3$ (voir par exemple US-A-3.855.342, US-A-3.855.343, US-A-3.862.258 et US-A-3.879.489), les résines perfluorées de type NAFION (voir par exemple US-A-4.056.578 et US-A-4.038.213), les acides de Lewis et/ou de Bronsted déposés sur divers supports inorganiques (voir par exemple US-A-3.975.299, US-A-3.852.371 et US-A-3.979.476), les alumines chlorées (voir par exemple US-A-3.240.840, US-A-3.523.142, US-A-3.607.859, US-A-3.523.142, US-A-4.066.716, US-A-4.083.800 et US-A-4.066.716), les graphites intercalés par des acides de Lewis et/ou de Bronsted (voir par exemple US-A-4.083.885, US-A-4.116.880, US-A-4.128.596 et US-A-3.976.714) et les anions déposés sur supports oxydes tels que $ZrO_2/SO_4$ (voir par exemple J-01288329, J-011245854-A, J-01245953, J-61242641-A et J-61242641). Ces solides conduisent à la production d'isoparaffines ramifiées mais souffrent de plusieurs défauts majeurs, parmi lesquels on peut citer l'utilisation d'un rapport molaire isoparaffine(s)-oléfine(s) souvent très élevé pour limiter l'importance des réactions secondaires et la faible stabilité dans le temps de l'activité catalytique (inhibition du catalyseur par dépôt d'oligomères insaturés) ; ces catalyseurs doivent alors être fréquemment régénérés. De plus, la faible acidité de certains solides acides, tels que les tamis moléculaires par exemple, impose l'utilisation de températures de réaction élevées ce qui est préjudiciable à l'obtention d'hydrocarbures d'indice d'octane élevé.

L'invention concerne un nouveau catalyseur permettant d'obtenir des composés paraffiniques à haut degré de ramification et haut indice d'octane par alkylation de l'isobutane et/ou de l'isopentane, de préférence de l'isobutane, au moyen d'au moins une oléfine comprenant de 2 à 6 atomes de carbone par molécule.

Le catalyseur de la présente invention renferme un support poreux organique ou minéral et au moins un mélange constitué d'au moins un halogénure de composé choisi dans le groupe formé par le bore et l'aluminium, c'est-à-dire d'au moins un halogénure d'aluminium et/ou de bore, de préférence un halogénure d'aluminium, et d'au moins un composé choisi dans le groupe formé par les halogénures d'ammonium et les halohydrates d'amine, c'est-à-dire au moins un halogénure d'ammonium et/ou halohydrate d'amine, de préférence au moins un halogénure d'ammonium et/ou un chlorhydrate d'amine et/ou un bromhydrate d'amine, le support étant imprégné par au moins un mélange constitué d'au moins un halogénure d'aluminium et/ou de bore et d'au moins un halogénure d'ammonium et/ou halohydrate d'amine. Ledit mélange est un complexe ionique non miscible à la phase hydrocarbure communément appelé "sel fondu". Il peut être obtenu liquide à température ambiante, mais aussi à température plus élevée de préférence inférieure à 150°C et de manière encore plus préférée inférieure à 100°C. Dans tous les cas, il est liquide lors de son imprégnation sur le support.

Le support est choisi parmi les supports poreux organiques ou minéraux connus de l'homme du métier. Parmi les supports organiques que l'on peut utiliser, on peut citer à titre d'exemples non limitatifs : les résines macroréticulaires, les résines perfluorées et le charbon. Parmi les supports minéraux que l'on peut utiliser on peut citer les oxydes tels que par exemple $SiO_2$, $ZrO_2$, $TiO_2$, $SnO_2$, $Al_2O_3$, $Fe_2O_3$, $HfO_2$ et toute combinaison de deux au moins de ces composés ($ZrO_2$-$TiO_2$ par exemple). Le support préféré est la silice.

La surface spécifique du support que l'on peut utiliser doit généralement être comprise entre 0,01 et 1500 $m^2/g$, de préférence entre 0,01 et 150 $m^2/g$ et de manière encore plus préférée entre 0,01 et 50 $m^2/g$. Le volume poreux total du

support doit généralement être compris entre 0,005 et 1,5 $cm^3/g$, de préférence entre 0,005 et 1 $cm^3/g$ et de manière encore plus préférée entre 0,005 et 0,8 $cm^3/g$. Lors de l'imprégnation dudit support par au moins un mélange constitué d'au moins un halogénure d'aluminium et/ou de bore et d'au moins un halogénure d'ammonium et/ou halohydrate d'amine, le(s)dit(s) mélange(s) doi(ven)t généralement occuper une fraction du volume poreux total du support oxyde comprise entre 5 et 100 %. Le catalyseur ainsi obtenu est caractérisé par une surface spécifique généralement comprise entre 0,01 et 500 $m^2/g$, de préférence entre 0,01 et 150 $m^2/g$ et de manière encore plus préférée entre 0,01 et 40 $m^2/g$.

Les halogénures d'aluminium et/ou de bore plus particulièrement utilisables dans la présente invention sont les trifluorures, les trichlorures et/ou les tribromures d'aluminium et/ou de bore tels que par exemple $AlCl_3$, $AlBr_3$, $BCl_3$ ou $BBr_3$, de préférence le trifluorure, le trichlorure et/ou le trifluorure d'aluminium : $AlCl_3$, $AlBr_3$ et $AlF_3$. Ils peuvent être mis en oeuvre purs ou en mélange, par exemple, dans des proportions variables allant de 99% de l'un des halogénures jusqu'à 99% de l'autre. On peut ainsi mélanger $AlCl_3$ avec $AlBr_3$, $AlCl_3$ avec $BCl_3$ ou $AlBr_3$ avec $BBr_3$.

Les halogénures d'ammonium quaternaires, plus particulièrement utilisables selon l'invention, acycliques ou faisant partie d'un cycle répondent aux formules générales suivantes :

$$R_1R_2R_3R_4N^+ X^- \qquad (I)$$

$$R_1R_2N^+=CR_3R_4 X^- \qquad (II)$$

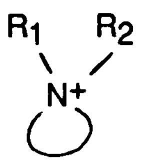 X- (III)

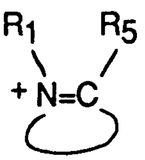 X- (IV)

dans lesquelles $R_1$, $R_2$, $R_3$, $R_4$ ou $R_5$ identiques ou différents, représentent des restes hydrocarbyles, par exemple alkyl, cycloalkyl, aryl, aralkyl ou alkylaryl, $R_5$ pouvant être également l'hydrogène ou un reste hydrocarbyle substitué comprenant par exemple au moins un atome différent de l'hydrogène et du carbone, comme l'azote. Il est encore possible qu'un radical puisse unir deux molécules dont au moins l'une d'entre elles est telle que ci-dessus comme, par exemple, $R_1R_2N+=CR_3-R_6-CR_3=N+R_1R_2 (X-)_2$, $R_6$ étant un radical et pouvant encore être un reste alkylène ou encore phénylène. Les cycles des formules (III) et (IV) sont constitués de 4 à 19 atomes, de préférence de 5 à 6 atomes, et peuvent comprendre, outre l'azote de l'ammonium quaternaire, des atomes de carbone ou éventuellement d'autres atomes d'azote. Ces cycles peuvent être aromatiques ou non. Les cycles des formules (III) et (IV) peuvent être condensés avec d'autres cycles et porter des substituants hydrocarbonés tels que ceux définis pour $R_1$, des fonctions amines, des atomes de fluor, de chlore ou de brome.

Parmi les groupements R1, R2, R3, R4 et R5 on peut mentionner les radicaux, méthyl, éthyl, propyl, isopropyl, butyl, secondaire butyl, tertiaire butyl, amyl, méthylène, éthylidène, phényl ou benzyl ; $R_6$ peut être un groupement méthylène, éthylène, propylène ou phénylène. Les cycles tels que décrits dans la formule (IV) sont représentés par la famille des pyridines, des imidazoles, des triazines, des pyrazoles, des pyrimidines, des triazoles.

Dans ces formules (I) à (IV), X- représente un ion halogénure, par exemple l'ion bromure ou l'ion chlorure.

Comme exemple de sels d'ammonium utilisables selon l'invention on peut citer plus particulièrement les sels d'imidazolium et de pyridinium tels que les chlorures et bromures de N, N'-dihydrocarbyl imidazolium et de N-hydrocarbylpyridinium, notamment les chlorures de méthyl-1 éthyl-3 imidazolium, le bromure de méthyl-1 butyl-3 imidazolium, le chlorure de phényl-1 isopropyl-3 imidazolium, le chlorure de N-méthylpyridinium, le bromure de N-isopropylpyridinium, le chlorure de N-benzylpyridinium.

Les halohydrates d'amines plus particulièrement utilisables selon l'invention comportent une mole d'acide halohydrique par mole d'amine, mais peuvent également comporter au moins deux moles d'acide halohydrique par mole d'amine, et de préférence deux moles d'acide halohydrique par mole d'amine. Il est aussi possible d'utiliser des mélanges d'halohydrates à au moins une mole d'acide halohydrique, par exemple des mélanges d'halohydrates à une et deux moles d'acide halohydrique.

Lesdits halohydrates d'amines dérivent d'amines ou de diamines cycliques ou d'amines faisant partie d'un cycle qui comportent au moins deux atomes d'azote, et ils répondent aux formules générales suivantes :

$$R_1R_2R_3NH^+\ X^- \qquad (V)$$

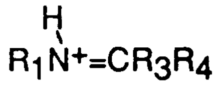 $X^-$ (VI)

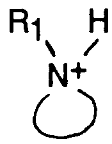 $X^-$ (VII)

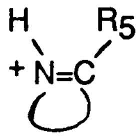 $X^-$ (VIII)

dans lesquelles $R_1$, $R_2$, $R_3$, $R_4$ ou $R_5$ identiques ou différents, représentent des restes hydrocarbyles, par exemple alkyl, cycloalkyl, aryl, aralkyl ou alkylaryl, ou bien encore représentent l'hydrogène, $R_5$ pouvant être également un reste hydrocarbyle substitué comprenant par exemple au moins un atome différent de l'hydrogène et du carbone, comme l'azote. Pour obtenir un halohydrate d'amine, il est encore possible qu'un radical puisse unir deux molécules dont au moins l'une d'entre elles est telle que ci-dessus comme, par exemple, $R_1HN^+=CR_3-R_6-CR_3=N^+R_1R_2\ (X^-)_2$ ou $R_1HN^+=CR_3-R_6-CR_3=N+R_1H\ (X^-)_2$ ,$R_6$ étant un radical et pouvant être un reste alkylène ou encore phénylène. Les cycles des formules (VII) et (VIII) sont constitués de 4 à 19 atomes, de préférence de 5 à 6 atomes, et peuvent comprendre, outre au moins deux atomes d'azote de l'amine, des atomes de carbone ou éventuellement d'autres atomes d'azote ; ces cycles peuvent être aromatiques ou non. Les cycles des formules (VII) et (VIII) peuvent être condensés avec d'autres cycles et porter des substituants hydrocarbonés tels que ceux définis pour $R_1$, des fonctions amines, des atomes de fluor, de chlore ou de brome.

Parmi les groupements R1, R2, R3, R4 et R5 on peut mentionner l'atome d'hydrogène, les radicaux, méthyl, éthyl, propyl, isopropyl, butyl, secondaire butyl, tertiaire butyl, amyl, méthylène, éhtylidène, phényl ou benzyl ; $R_6$ peut être un groupement méthylène, éthylène, propylène ou phénylène. Les cycles tels que décrits dans la formule (VIII) sont représentés par les familles des pyridines, des imidazoles, des triazines, des pyrazoles, des pyrimidines, des triazoles.

Dans ces formules(V) à (VIII), X- représente un ion halogénure, de préférence l'ion bromure ou l'ion chlorure.

Comme exemple de chlorhydrates ou de bromhydrates d'amines qui sont utilisables selon l'invention, on peut citer plus particulièrement, les chlorhydrates et bromhydrates de 2-picolinium, 3-picolinium ou 4-picolinium, les chlorhydrates et bromhydrates de pipéridinium, de pipérazinium,de morpholinium, et de méthyl-2 pyrazinium.

Pour des composants de nature déterminée les proportions relatives d'halogénure(s) d'aluminium et/ou de bore et d'halogénure(s) d'ammonium et/ou halohydrate(s) d'amines déterminent à la fois l'acidité (ou la basicité) du milieu et son point de cristallisation. Quand la fraction molaire d'halogénure(s) d'aluminium et/ou de bore est inférieure à 0,5, le mélange est très peu acide, voire basique. Si elle est supérieure à 0,5, le milieu est d'autant plus acide que cette fraction molaire est grande. On dispose donc d'une gamme d'acidité très étendue que l'on peut faire varier en fonction de la réactivité de l'oléfine, ce qui a l'avantage de minimiser les réactions parasites d'oligomérisation en maximisant la vitesse de réaction.

Le procédé de préparation du catalyseur selon l'invention comprend deux étapes. Dans une première étape le support est calciné à une température généralement supérieure à 50 °C, de préférence supérieure à 80 °C et de manière encore plus préférée comprise entre 200 et 600°C, par exemple égale à environ 500 °C. La durée de cette étape de calcination est habituellement comprise entre 10 minutes et 50 heures. La calcination peut être effectuée en présence d'air ou de mélange air/azote, le débit étant généralement compris entre 0,001 et 10 l/h/g. La seconde étape consiste en l'imprégnation du produit calciné par au moins un mélange constitué d'au moins un halogénure d'aluminium et/ou de bore et d'au moins un halogénure d'ammonium et/ou halohydrate d'amine. La température d'imprégnation est telle que le(s)dit(s) mélange(s) est(sont) liquide(s) lors de l'imprégnation du support. Pour réaliser cette étape on peut utiliser toutes les techniques bien connues de l'homme du métier. Le solide ainsi obtenu est alors conservé à l'abri de

l'humidité. Le catalyseur selon la présente invention est ainsi constitué d'un support poreux organique ou minéral imprégné par au moins un mélange constitué d'au moins un halogénure d'aluminium et/ou de bore et d'au moins un halogénure d'ammonium et/ou halohydrate d'amine.

Le catalyseur selon la présente invention peut être utilisé pur ou dilué avec divers matériaux présentant peu d'activité catalytique dans la réaction considérée comme par exemple la silice, l'alumine, la magnésie ou encore diverses argiles telles que par exemple la bentonite, la montmorillonite ou le kaolin.

Ledit catalyseur est avantageusement mis en oeuvre dans un procédé où l'oléfine et/ou un mélange d'oléfines est introduit(e) dans le réacteur en phase liquide et en mélange avec l'isoparaffine et/ou un mélange d'isoparaffines. Le catalyseur peut être mis en oeuvre en lit fixe, lit mobile ou lit fluide, ou encore en suspension dans la phase liquide des réactifs soumise à une agitation efficace.

Le mélange isoparaffine(s)-oléfine(s) peut être introduit dans le réacteur à une vitesse spatiale horaire, exprimée en poids d'oléfine introduite par unité de poids du catalyseur et par heure, généralement comprise entre 0,001 et 10 $h^{-1}$ et de préférence comprise entre 0,002 et 2 $h^{-1}$. Ledit mélange peut aussi être réalisé à l'intérieur du réacteur. Dans tous les cas le mélange ainsi constitué est dans le réacteur dans des conditions de pression et de température telles que le mélange d'hydrocarbures reste liquide sur le catalyseur.

La température de réaction est généralement comprise entre -50 et 150 °C, mais les performances catalytiques sont améliorées lorsque la température de réaction est inférieure à 5°C, de préférence inférieure à 0 °C et de manière encore plus préférée inférieure à -5 °C. La pression du réacteur doit être suffisante pour maintenir les hydrocarbures à l'état liquide dans le réacteur quelle que soit la température.

Afin de limiter les réactions secondaires, on utilise généralement un excès d'isoparaffine(s) par rapport à l' (aux) oléfine(s). A titre d'exemple, dans le cas de l'alkylation de l'isobutane par un butène, l'isobutane est introduit pur dans la charge ou sous la forme d'un mélange de butanes contenant par exemple au moins 40 % d'isobutane. De plus, on peut introduire un butène pur ou encore un mélange de butènes isomères. Dans tous les cas, le rapport molaire isobutane/butène(s) dans la charge est généralement compris entre 1 et 100, de préférence entre 3 et 50 et de manière souvent préférée entre 5 et 10.

Les produits de la réaction peuvent être contrôlés régulièrement par mesure de l'indice de brome, par exemple selon le projet de Norme Française Pr. M. 07.071 de mars 1969.

Lorsque la nature du catalyseur et les conditions de travail du catalyseur sont judicieusement choisies (et en particulier la température), le catalyseur selon l'invention permet la production de produits d'alkylation de paraffine(s) par au moins une oléfine qui sont intéressants comme carburants pour les moteurs et constituants d'essence et qui comprennent par exemple au moins 60 % (en moles) de paraffines possédant 8 atomes de carbone par molécule et moins de 1 % (en moles) de composés non saturés, les paraffines possédant 8 atomes de carbone par molécule étant composées de 70 à 98 % (en moles) de triméthylpentanes.

Un autre avantage du catalyseur selon la présente invention est la possibilité d'alkyler, à basse température, l'isobutane avec des mélanges d'oléfines comportant de 2 à 6 atomes de carbone par molécule, où la proportion d'oléfines comportant au moins 5 atomes de carbone par molécule est très importante (au moins 10% en poids, de préférence au moins 40 % en poids).

Les exemples suivants illustrent l'invention sans toutefois en limiter la portée.

## Exemple 1

Préparation du catalyseur

On prépare un complexe ionique de normalité 0,55 N de la façon suivante : à 10,3 g de $AlCl_3$ fraîchement sublimé on ajoute lentement 10,9 g de méthyl butyl imidazolium chlorure, en agitant à l'aide d'un barreau magnétique. La température est maintenue constante à 0°C. On obtient ainsi un mélange liquide à température ambiante, très faiblement coloré. Ce sel est conservé à l'abri de l'humidité.

On active 14 g de silice macroporeuse, de surface spécifique égale à 27 $m^2/g$ par calcination sous air pendant 4 heures à 500°C. Le solide ainsi activé est conservé sous argon. On procède alors à une imprégnation à sec de 10 g du solide calciné par 4,5 g du mélange préparé comme indiqué ci-dessus. Le catalyseur ainsi obtenu contient 4,5 g de mélange et 10 g de silice et est conservé sous argon à -18 °C.

Alkylation de l'isobutane par le butène-2

On introduit 12 g du catalyseur préparé selon la méthode décrite ci-dessus dans un réacteur en verre du type Fischer & Porter d'un volume de 360 ml préalablement purgé sous débit d'argon. Le réacteur contenant le catalyseur est alors fermé puis mis sous vide primaire, puis il est refroidi à la température de -20°C.

72 $cm^3$ d'isobutane sont alors ajoutés dans le réacteur contenant le catalyseur sous agitation, ledit réacteur étant immergé dans un bain froid à -20 °C. On laisse sous agitation le système catalyseur + isobutane pendant une durée de

30 minutes afin d'homogénéiser la température.

On ajoute régulièrement 13,16 $cm^3$ de butène-2 pendant une durée totale de 6 heures, la température du réacteur étant maintenue à -15° C pendant toute la durée de l'injection.

Après réaction, la phase hydrocarbure est soutirée du réacteur, l'isobutane est évaporé lentement. On recueille l'alkylat qui est analysé par chromatographie en phase vapeur ; sa composition pondérale est donnée ci-dessous. La conversion de l'oléfine est de 100 %. La composition de l'alkylat est reportée dans le tableau 1 ci-dessous.

Tableau 1

| | |
|---|---|
| $iC_5$ | 1,0 |
| $C_6$ | 0,9 |
| $C_7$ | 1,6 |
| $C_8$ | 93,8 |
| $C_9$ | 1,2 |
| $C_9$+ | 1,5 |

La fraction $C_8$ contient 94,6% poids de triméthylpentanes, l'indice d'octane recherche calculé est de 99.

**Exemple 2**

On répète le test catalytique d'alkylation de l'isobutane par le butène-2 avec le même catalyseur que celui de l'exemple 1, et on effectue une série de tests à de températures différentes. Les résultats sont rassemblés dans le tableau 2 ci-dessous.

Tableau 2

| T (°C) | $C_5$-$C_7$ | $C_8$ | $C_9$+ | RON |
|---|---|---|---|---|
| -20 | 2,8 | 92,3 | 4,9 | 99,3 |
| -10 | 2,1 | 97 | 0,9 | 98,4 |
| 0 | 6,4 | 86,7 | 6,9 | 96,4 |

Ce tableau met en évidence l'intérêt qu'il y a à travailler à basse température.

**Exemple 3 (comparatif)**

Préparation du catalyseur

On prépare un complexe ionique de normalité 0,67 N de la façon suivante : à une suspension de 24,8 g d'$AlCl_3$ fraîchement sublimé, dans l'heptane on ajoute lentement 11,5 g de chlorhydrate de pyridinium, en agitant à l'aide d'un barreau magnétique. On obtient ainsi un mélange, liquide à température ambiante, très faiblement coloré. Ce mélange est conservé sous argon à l'abri de l'humidité.

On active 13 g de silice macroporeuse, de surface spécifique égale à 27 $m^2$/g par calcination sous air pendant 4 heures à 500°C. Le solide ainsi activé est conservé sous argon. On procède alors à une imprégnation à sec de 13 g du solide calciné par 8,8 g de mélange préparé comme indiqué ci-dessus. Le catalyseur ainsi obtenu contient 8,8 g de mélange et 13 g de silice et est conservé sous argon à -18 °C.

Alkylation de l'isobutane par le butène-2

On introduit le catalyseur préparé selon la méthode décrite ci-dessus dans un réacteur en verre du type Fischer & Porter d'un volume de 360 ml préalablement purgé sous débit d'argon. Le réacteur contenant le catalyseur est alors fermé puis mis sous vide primaire, puis il est refroidi à la température de -15°C.

80,6g d'isobutane sont alors ajoutés dans le réacteur contenant le catalyseur sous agitation, ledit réacteur étant

immergé dans un bain froid à -15 °C. On laisse sous agitation le système catalyseur + isobutane pendant une durée de 30 minutes afin d'homogénéiser la température.

On ajoute régulièrement 16,5 g de butène-2 pendant une durée totale de 3 heures, la température du réacteur étant maintenue à -15 °C pendant toute la durée de l'injection.

Après réaction, la phase hydrocarbure est soutirée du réacteur, l'isobutane est évaporé lentement. On recueille l'alkylat qui est analysé par chromatographie en phase vapeur ; sa composition pondérale est donnée ci-dessous. La conversion de l'oléfine est de 100 %. La composition de l'alkylat, en % poids, est reportée dans le tableau 3 ci-dessous.

Tableau 3

| | |
|---|---|
| $iC_5$ | 0,9 |
| $C_6$ | 1,1 |
| $C_7$ | 1,3 |
| $C_8$ | 93,3 |
| $C_9$ | 1,2 |
| $C_9$+ | 2,2 |

La fraction $C_8$ contient 93,2% poids de triméthylpentanes, l'indice d'octane recherche calculé est de 98,6.

**<u>Exemple 4</u> (comparatif)**

On répète le test catalytique d'alkylation de l'isobutane par le butène-2 avec le même catalyseur que celui de l'exemple 3, et on effectue une série de tests à différentes températures. Les résultats sont rassemblés dans le tableau 4 ci-dessous.

Tableau 4

| T (°C) | $C_5$-$C_7$ | $C_8$ | $C_9$+ | RON |
|---|---|---|---|---|
| -20 | 2,1 | 95,2 | 2,7 | 99,5 |
| -10 | 4,0 | 89,9 | 4,9 | 97,3 |
| 0 | 5,9 | 87,8 | 7,3 | 96,1 |

Ce tableau met en évidence l'intérêt qu'il y a à travailler à basse température.

**<u>Exemple 5</u> (compartif)**

<u>Préparation du catalyseur</u>

On prépare un complexe ionique de normalité 0,67 N de la façon suivante : à 28,8 g de $AlCl_3$ fraîchement sublimé, dans l'heptane on ajoute lentement 12,9 g de chlorhydrate de 4-picolinium, en agitant à l'aide d'un barreau magnétique. On obtient ainsi un mélange, liquide à température ambiante,très faiblement coloré. Ce mélange est conservé sous argon à l'abri de l'humidité.

On active 13 g de silice macroporeuse, de surface spécifique égale à 27 $m^2$/g par calcination sous air pendant 4 heures à 500°C. Le solide ainsi activé est conservé sous argon. On procède alors à une imprégnation à sec de 13 g du solide calciné par 8,5 g de mélange préparé comme indiqué ci-dessus. Le catalyseur ainsi obtenu contient 8,5 g de mélange et 13 g de silice et est conservé sous argon à -18 °C.

<u>Alkylation de l'isobutane par le butène-2</u>

On introduit le catalyseur préparé selon la méthode décrite ci-dessus dans un réacteur en verre du type Fischer & Porter d'un volume de 360 ml préalablement purgé sous débit d'argon. Le réacteur contenant le catalyseur est alors fermé puis mis sous vide primaire, puis il est refroidi à la température de -15°C. Le même test catalytique, d'alkylation

de l'isobutane par le butène-2, que celui réalisé dans l'exemple 3 à -15°C est alors répété. Les résultats sont rassemblés dans le tableau 5 ci-après.

Tableau 5

| | |
|---|---|
| $iC_5$ | 1,1 |
| $C_6$ | 0,8 |
| $C_7$ | 1,4 |
| $C_8$ | 93,6 |
| $C_9$ | 1,0 |
| $C_9$+ | 2,1 |

La fraction $C_8$ contient 94,0% poids de triméthylpentanes, l'indice d'octane recherche calculé est de 98,9.

**Revendications**

1. Catalyseur comprenant un support poreux organique ou minéral et au moins un mélange constitué d'au moins un halogénure de composé choisi dans le groupe formé par l'aluminium et le bore et d'au moins un composé choisi dans le groupe formé par les halogénures d'ammonium et les halohydrates d'amine, ledit composé n'étant pas choisi dans les halogénures de base azotée cyclique comprenant qu'un seul atome d'azote dans un cycle, ledit mélange étant un complexe ionique, non miscible à la phase hydrocarbure, liquide lors de son imprégnation sur le support, et le support ayant été imprégné par ledit mélange.

2. Catalyseur selon la revendication 1 tel que 'halogénure de composé choisi dans le groupe formé par l'aluminium et le bore est choisi dans le groupe formé par $AlCl_3$, $AlBr_3$ et $AlF_3$.

3. Catalyseur selon l'une des revendications 1 ou 2 tel que ledit composé est un halogénure d'ammonium quaternaire dont la formule générale est l'une des formules suivantes :

$$R_1R_2R_3R_4N^+ X^- \quad (I)$$

$$R_1R_2N^+{=}CR_3R_4 X^- \quad (II)$$

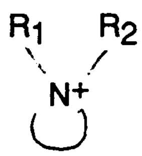

X- (III)

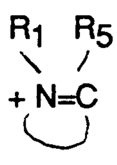

X- (IV)

dans lesquelles $R_1$, $R_2$, $R_3$, $R_4$ ou $R_5$ représentent des restes hydrocarbyles, $R_5$ pouvant être en outre de l'hydrogène, et $X^-$ un ion halogénure.

4. Catalyseur selon l'une des revendications 1 ou 2 dans lequel ledit composé est un chlorure ou un bromure de N,N' dialkyl imidazolium.

5. Catalyseur selon l'une des revendications 1 ou 2 tel que ledit composé est un halohydrate d'amine dont la formule générale est l'une des formules suivantes :

$$R_1R_2R_3NH^+ X^- \quad (V)$$

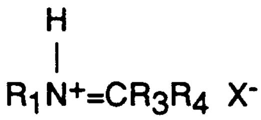

(VI)

dans lesquelles $R_1$, $R_2$, $R_3$ ou $R_4$, identiques ou différents, représentent des restes hydrocarbyles ou l'hydrogène, et $X^-$ représente un ion halogénure.

6. Catalyseur selon l'une des revendications 1 ou 2 tel que ledit composé est un halohydrate d'amine choisi dans le groupe formé par les chlorhydrates et bromhydrates de pipérazinium et les chlorhydrates et bromhydrates de méthyl-2 pyrazinium.

7. Procédé de préparation d'un catalyseur selon l'une des revendications 1 à 6 tel que le support est calciné puis imprégné par ledit mélange.

8. Procédé d'alkylation catalytique d'au moins un élément choisi dans le groupe formé par l'isobutane et l'isopentane par au moins un élément choisi dans le groupe formé par les oléfines comprenant de 2 à 6 atomes de carbone par molécule, par un catalyseur selon l'une des revendications 1 à 6 ou préparé selon la revendication 7

9. Procédé selon la revendication 8 dans laquelle la température de la réaction est inférieure à 5°C.

10. Procédé d'alkylation catalytique d'au moins un élément choisi dans le groupe formé par l'isobutane et l'isopentane par au moins un élément choisi dans le groupe formé par les oléfines comprenant de 2 à 6 atomes de carbone par molécule, ledit procédé étant selon les revendications 8 ou 9 ou utilisant un catalyseur selon l'une des revendications 1 à 6 ou préparé selon la revendication 7, ledit procédé étant tel que le catalyseur est mis en oeuvre en lit mobile.

11. Procédé d'alkylation catalytique d'au moins un élément choisi dans le groupe formé par l'isobutane et l'isopentane par au moins un élément choisi dans le groupe formé par les oléfines comprenant de 2 à 6 atomes de carbone par molécule, ledit procédé étant selon les revendications 8 ou 9 ou utilisant un catalyseur selon l'une des revendications 1 à 6 ou préparé selon la revendication 7, ledit procédé étant tel que le catalyseur est mis en oeuvre en lit fluide.

12. Procédé d'alkylation catalytique d'au moins un élément choisi dans le groupe formé par l'isobutane et l'isopentane par au moins un élément choisi dans le groupe formé par les oléfines comprenant de 2 à 6 atomes de carbone par molécule, ledit procédé étant selon les revendications 8 ou 9 ou utilisant un catalyseur selon l'une des revendications 1 à 6 ou préparé selon la revendication 7, ledit procédé étant tel que le catalyseur est mis en oeuvre en suspension dans la phase liquide des réactifs soumise à une agitation efficace.

## Claims

1. Catalyst comprising an organic or mineral porous support and at least one mixture constituted by at least one halide of a compound chosen from within the group formed by aluminium and boron and at least one compound chosen from within the group formed by ammonium halides and amine hydrohalides said compound being not chosen amoung the cyclic nitrogen bases containing only one nitrogen atom within the ring, said mixture being an ionic complexe, immiscible with the hydrocarbon phase, liquid during its impregnation on the support, and the support having been impregnated by said mixture.

2. Catalyst according to claim 1 such that the halide of the compound chosen from within the group formed by aluminium and boron is chosen from among $AlCl_3$, $AlBr_3$ and $AlF_3$.

3. Catalyst according to one of the claims 1 or 2, such that said compound is a quaternary ammonium halide, whose general formula is one of the following formulas:

$$R_1R_2R_3R_4N^+ X^- \quad (I)$$

$$R_1R_2N^+{=}CR_3R_4 X^- \quad (II)$$

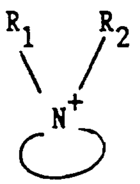

$X^-$ (III)

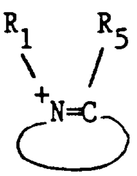

$X^-$ (IV)

in which $R_1$, $R_2$, $R_3$, $R_4$ or $R_5$ represent hydrocarbyl residues and $R_5$ can also be hydrogen, whilst $X^-$ is a halide ion.

4. Catalyst according to one of the claims 1 to 3, wherein said compound is a chloride or bromide of N,N'-dialkyl imidazolium.

5. Catalyst according to one of the claims 1 or 2, such that the said compound is an amine hydrohalide, whose general formula is one of the following formulas:

$$R_1R_2R_3 NH^+ X^- \quad (V)$$

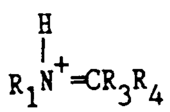

$X^-$ (VI)

in which $R_1$, $R_2$, $R_3$ or $R_4$, which are the same or different, represent hydrocarbyl residues or hydrogen and $X^-$ represents a halide ion.

6. Catalyst according to one of the claims 1 or 2, such that the said compound is an amine hydrohalide chosen from within the group formed by hydrochlorides and hydrobromides of piperazinium and hydrochlorides and hydrobromides of 2-methyl pyrazinium.

7. Process for the preparation of a catalyst according to one of the claims 1 to 6, such that the support is calcined and then impregnated by the said mixture.

8. Process for the catalytic alkylation of at least one element chosen from within the group formed by isobutane and isopentane by at least one element chosen from within the group formed by olefins having 2 to 6 carbon atoms per molecule, using a catalyst according to one of the claims 1 to 6 or prepared according to claim 7.

9. Process according to claim 8, wherein the reaction temperature is below 5°C.

10. Process for the catalytic alkylation of at least one element chosen from within the group formed by isobutane and isopentane by at least one element chosen from within the group formed by olefins having 2 to 6 carbon atoms per

molecule, said process being according to one of the claims 8 or 9 or using a catalyst according to one of the claims 1 to 6 or prepared according to claim 7, said process being such that the catalyst is used in a mobile bed.

11. Process for the catalytic alkylation of at least one element chosen from within the group formed by isobutane and isopentane by at least one element chosen from within the group formed by olefins having 2 to 6 carbon atoms per molecule, said process being according to one of the claims 8 or 9 or using a catalyst according to one of the claims 1 to 6 or prepared according to claim 7, said process being such that the catalyst is used in a fluid bed.

12. Process for the catalytic alkylation of at least one element chosen from within the group formed by isobutane and isopentane by at least one element chosen from within the group formed by olefins having 2 to 6 carbon atoms per molecule, said process being according to one of the claims 8 or 9 or using a catalyst according to one of the claims 1 to 6 or prepared according to claim 7, said process being such that the catalyst is used suspended in the liquid phase of the reagents subject to an effective stirring.

**Patentansprüche**

1. Katalysator, umfassend einen organischen oder mineralischen porösen Träger und wenigstens ein Gemisch, das aus wenigstens einer Halogenverbindung, ausgewählt aus der Gruppe, die durch Aluminium und Bor gebildet ist und wenigstens einer Verbindung, ausgewählt aus der Gruppe, die durch die Ammoniumhalogenide und die Aminhalohydrate gebildet ist, zusammengesetzt ist, wobei die Verbindung nicht aus den Halogeniden von cyclischen Stickstoffbasen ausgewählt ist, welche kein einziges Stickstoffatom im Ring aufweisen, wobei die Mischung ein nicht mit der Kohlenwasserstoffphase mischbarer ionischer Komplex ist, der während der Imprägnierung auf dem Träger flüssig ist und der Träger mit der Mischung imprägniert wird.

2. Katalysator nach Anspruch 1, dadurch gekennzeichnet, daß die Halogenverbindung, ausgewählt aus der durch Aluminium und Bor gebildeten Gruppe aus der Gruppe ausgewählt ist, die durch $AlCl_3$, $AlBr_3$ und $AlF_3$ gebildet ist.

3. Katalysator nach einem der Ansprüche 1 oder 2, dadurch gekennzeichnet, daß die Verbindung ein quaternäres Ammoniumhalogenid ist, dessen allgemeine Formel einer der folgenden Formeln entspricht:

$$R_1R_2R_3R_4N^+ X^- \quad (I)$$

$$R_1R_2N^+{=}CR_3R_4 X^- \quad (II)$$

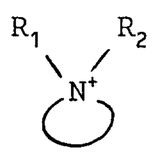

$X^-$ (III)

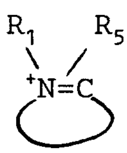

$X^-$ (IV)

worin $R_1$, $R_2$, $R_3$, $R_4$ und $R_5$ Kohlenwasserstoffreste darstellen, $R_5$ des weiteren ein Wasserstoffatom sein kann und $X^-$ ein Halogenion ist.

4. Katalysator nach einem der Ansprüche 1 oder 2, worin die Verbindung eine N,N'-Dialklyimidazoliumchlorid oder -bromid ist.

5. Katalysator nach einem der Ansprüche 1 oder 2, dadurch gekennzeichnet, daß die Verbindung eine Aminhalohydrat ist, dessen allgemeine Formel einer der folgenden Formeln entspricht:

$$R_1R_2R_3NH^+ X^- \quad (V)$$

$$\begin{array}{l} \quad\ \ H \\ \quad\ \ | \\ R_1N^{+}{=}CR_3R_4\ \ X^{-} \end{array} \qquad (VI)$$

worin $R_1$, $R_2$, $R_3$ oder $R_4$, welche gleich oder verschieden voneinander sind, Kohlenwasserstoffrest oder ein Wasserstoffatom darstellen und $X^-$ ein Halogenion ist.

6. Katalysator nach einem der Ansprüche 1 oder 2, dadurch gekennzeichnet, daß die Verbindung ein Aminhalohydrat ist, das aus der Gruppe ausgewählt ist, die durch die Chlorhydrate und Bromhydrate von Piperazinium und die Chlorhydrate und Bromhydrate von Methyl-2-Pyrazinium gebildet ist.

7. Verfahren zur Herstellung eines Katalysators, nach einem der Ansprüche 1 bis 6, dadurch gekennzeichnet, daß der Träger kalziniert und dann durch die Mischung imprägniert wird.

8. Verfahren zur katalytischen Alkylierung wenigstens eines Grundstoffes, ausgewählt aus der Gruppe, die durch das Isobutan und das Isopentan gebildet ist, mit wenigstens einem Grundstoff, ausgewählt aus der Gruppe, die durch die 2 bis 6 Kohlenstoffatome pro Molekül umfassenden Olefine gebildet ist, durch einen Katalysator gemäß einem der Ansprüche 1 bis 6 oder hergestellt nach Anspruch 7.

9. Verfahren nach Anspruch 8, worin die Temperatur der Umsetzung unter 5°C liegt.

10. Verfahren zur katalytischen Alkylierung wenigstens eines Grundstoffes, ausgewählt aus der Gruppe, die durch das Isobutan und das Pentan gebildet ist, mit wenigstens einem Grundstoff, ausgewählt aus der Gruppe, die durch die 2 bis 6 Kohlenstoffatome pro Molekül umfassenden Olefine gebildet ist, wobei das Verfahren den Ansprüchen 8 oder 9 entspricht oder einen Katalysator nach einem der Ansprüche 1 bis 6 verwendet oder der Katalysator nach Anspruch 7 hergestellt ist, wobei das Verfahren dadurch gekennzeichnet ist, daß der Katalysator im Wirbelbett eingesetzt wird.

11. Verfahren zur katalytischen Alkylierung wenigstens eines Grundstoffes, ausgewählt aus der Gruppe, die durch das Isobutan und das Isopentan gebildet ist, mit wenigstens einem Grundstoff, ausgewählt aus der Gruppe, die durch die 2 bis 6 Kohlenstoffatome pro Molekül umfassenden Olefine gebildet ist, wobei das Verfahren den Ansprüchen 8 oder 9 entspricht oder einen Katalysator nach einem der Ansprüche 1 bis 6 verwendet oder der Katalysator nach Anspruch 7 hergestellt ist, wobei das Verfahren dadurch gekennzeichnet ist, daß der Katalysator im Fließbett eingesetzt wird.

12. Verfahren zur katalytischen Alkylierung wenigstens eines Grundstoffes, ausgewählt aus der Gruppe, die durch das Isobutan und das Isopentan gebildet ist, mit wenigstens einem Grundstoff, ausgewählt aus der Gruppe, die durch die 2 bis 6 Kohlenstoffatome pro Molekül umfassenden Olefine gebildet ist, wobei das Verfahren den Ansprüchen 8 oder 9 entspricht oder einen Katalysator nach einem der Ansprüche 1 bis 6 verwendet oder der Katalysator nach Anspruch 7 hergestellt, wobei das Verfahren dadurch gekennzeichnet ist, daß der Katalysator in Suspension in der flüssigen Phase der Reagenzien unter wirksamem Rühren eingesetzt wird.